Europäisches Patentamt

European Patent Office

Office européen des brevets

(19)

(11) **EP 0 656 010 B1**

(12) **FASCICULE DE BREVET EUROPEEN**

(45) Date de publication et mention
de la délivrance du brevet:
**13.06.2001 Bulletin 2001/24**

(51) Int Cl.⁷: **C07K 7/06**, A61K 39/42,
A61K 38/08, A61K 39/21

(21) Numéro de dépôt: **93917862.0**

(22) Date de dépôt: **10.08.1993**

(86) Numéro de dépôt international:
**PCT/FR93/00803**

(87) Numéro de publication internationale:
**WO 94/03487 (17.02.1994 Gazette 1994/05)**

(54) **Utilisation de peptide reconnu par une réponse immunitaire pour l'obtention de médicaments déstinés à l'induction de la supression immunitaire.**

Verwendung von Peptiden welche eine Immunantwort bewirken zur Herstellung von Medikamenten zur Induzierung der Immunsuppression.

Use of peptides causing a immune response for the preparation of medicaments to induce the suppression of the immune reaction.

(84) Etats contractants désignés:
**AT BE CH DE DK ES FR GB GR IE IT LI LU MC NL PT SE**

(30) Priorité: **10.08.1992 FR 9209884**
**12.02.1993 FR 9301628**

(43) Date de publication de la demande:
**07.06.1995 Bulletin 1995/23**

(73) Titulaire: **NEOVACS**
**F-75003 Paris (FR)**

(72) Inventeur: **NEOVACS**
**F-75003 Paris (FR)**

(74) Mandataire: **Rinuy, Santarelli**
**14, avenue de la Grande Armée,**
**B.P. 237**
**75822 Paris Cédex 17 (FR)**

(56) Documents cités:
**EP-A- 0 344 006        WO-A-90/00566**
**WO-A-90/03984**

• JOURNAL OF GENERAL VIROLOGY vol. 72, no. 8 , Août 1991 , COLCHESTER, GRANDE BRETAGNE pages 1913 - 1918 C. ÖRVELL ET AL. 'Immunological characterization of the human immunodeficiency virus type 1 reverse transcriptase protein by the use of monoclonal antibodies.'

• THE JOURNAL OF CLINICAL INVESTIGATION vol. 88, no. 3 , Septembre 1991 , NEW YORK, TATS-UNIS pages 876 - 884 J. BERZOFSKY ET AL. 'Construction of peptides encompassing multideterminant clusters of human immunodeficiency virus envelope to induce in vitro T cell responses in mice and humans of multiple MHC types.'

• SCANDINAVIAN JOURNAL OF IMMUNOLOGY vol. 35, no. 3 , Mars 1992 , OXFORD, GRANDE BRETAGNE pages 267 - 273 M. ZAITSEVA ET AL. 'Antibodies to MHC class II peptides are present in HIV-1-positive sera.'

• JOURNAL OF EXPERIMENTAL MEDICINE vol. 167, no. 3 , 1 Mars 1988 , NEW YORK, TATS-UNIS pages 914 - 923 H. GOLDING ET AL. 'Identification of homologous regions in human immunodeficiency virus 1 gp41 and human MHC class II-beta-1 domain. I. Monoclonal antibodies against the gp41-derived peptide and patients' sera react with native HLA class II antigens,...etc.'

• BIOMEDICINE AND PHARMACOTHERAPY vol. 47 , 1993 , PARIS, FRANCE pages 93 - 99 J. ZAGURY ET AL. 'HIV-1-induced immune suppression may result from autoimmune disorders including anti-SLWDQ autoantibodies.'

## Description

La présente demande concerne de nouveaux peptides, des anticorps dirigés contre ces peptides et des moyens de blocage de ces anticorps, leur application à titre de médicaments, des compositions pharmaceutiques et des méthodes d'utilisation de ces produits.

On a identifié des séquences peptidiques partagées par le virus VIH-1 et des molécules autologues (du soi), et contre lesquelles existe une réaction immunitaire chez les individus infectés par le VIH-1. Cette réaction immunitaire est donc auto-immune car dirigée contre des épitopes du soi, et permet d'expliquer le développement de la maladie et le dérèglement immunitaire observé au cours de l'infection par le VIH-1. La définition de ces peptides identifiés grâce à une stratégie informatique particulière permet le développement de stratégies prophylactiques et thérapeutiques contre le SIDA qui sont l'objet principal de la présente demande.

Le système immunitaire se présente sous deux aspects fonctionnels : l'immunité non spécifique et l'immunité spécifique, dite encore à mémoire. L'immunité non spécifique consiste en une première ligne de défense, capable d'arrêter la plupart des agents pathogènes avant que ne s'établisse une véritable infection. Les mécanismes de l'immunité spécifique entrent ensuite en action. Ils déclenchent une réaction dirigée spécifiquement contre le germe responsable, entraînant sa destruction. D'une manière générale et schématique, il existe deux grands types de réponse immunitaire spécifique : la réponse de type humoral qui est caractérisée par la production d'anticorps par les lymphocytes B et la réponse immunitaire à médiation cellulaire qui met en jeu des cellules effectrices, c'est à dire essentiellement les lymphocytes T8 (lymphocytes cytotoxiques). Ces réponses sont initialement activées par les cellules présentatrices d'antigènes et modulées par les cellules régulatrices, c'est à dire les lymphocytes T4 (lymphocytes T auxiliaires) et les lymphocytes T suppresseurs.

Dans ses très grandes lignes, la réponse immunitaire spécifique fonctionne comme suit:

- Les cellules présentatrices d'antigène appelées APC (monocytes, macrophages et lymphocytes B) capturent l'antigène, le digèrent et exposent des fragments de cet antigène "digéré" à leur surface, en association avec des molécules du complexe majeur d'histocompatibilité de classe I ou II. (CMH1 OU CMH2).
- Quand les lymphocytes T4 "voient" les fragments d'antigènes associés au complexe majeur d'histocompatibilité de classe II, ils prolifèrent, passent sous forme activée (et notamment synthétisent de l'II-2). Ce passage à l'état activé dépend essentiellement d'interactions moléculaires fines entre les 3 molécules CD4, CMH, et récepteurs des cellules T (TCR). Les lymphocytes T stimulent alors la prolifération des lymphocytes B producteurs d'anticorps et celle des lymphocytes T8 (lymphocytes cytotoxiques ou CTL) grâce à des facteurs tels que IL2 ou IFN gamma, le tout étant régulé par d'autres facteurs tels que l'IFN alpha.
- Les lymphocytes B produisent des anticorps qui vont interagir avec les antigènes circulants de manière à les neutraliser.
- Les lymphocytes T8 détruisent les cellules infectées quand ils reconnaissent les fragments d'antigènes associés au complexe majeur d'histocompatibilité de classe I (CMH1).

Comme on le voit la réponse immunitaire est faite d'une cascade d'événements impliquant :

- des facteurs sécrétés comme par exemple les cytokines (interleukines, interférons, etc...) et leurs récepteurs membranaires,
- des molécules de surface des cellules servant à leurs interactions comme par exemple la molécule CD4 ou le CMH 1 ou 2 ou les adhésines (LFA 1, etc..).
- des molécules de surface des cellules servant à induire des signaux de régulation cellulaire soit par liaison avec des facteurs sécrétés comme les interleukines ou les interférons, soit par phénomènes de transduction induits par les molécules de surface précitées. Parmi ces dernières, on peut citer le groupement proteique CD3 lié au TCR (récepteur des cellules T) qui est nécessaire à l'activation des cellules T induite par un antigène, ou encore la protéine Fas qui est une molécule essentielle de la régulation immunitaire car elle induit la mort programmée des cellules.

Toutes les molécules ainsi décrites qui participent au fonctionnement et à la régulation du système immunitaire sont dénommées dans la présente demande molécules immunorégulatrices.

La réponse immunitaire standard à un agent microbien quelconque peut donc être schématiquement résumée comme suit: l'intervention de l'immunité non spécifique est quasi-immédiate puis, si besoin est, elle est suivie dans les 48 à 72 heures de l'apparition de la réponse immunitaire spécifique. Une fois le pathogène évacué, il y a des mécanismes de régulation (comme par exemple la secrétion d'interféron alpha, l'expression de la protéine Fas et son activation pour préparer la mort programmée) qui permettent de contrôler la prolifération des cellules du système immunitaire induite par l'agent pathogène. Toutefois, il existe des exceptions à ce schéma directeur, en particulier lorsque l'agent microbien est un virus.

En effet, les virus surtout les virus à enveloppe et en particulier les rétrovirus, ont pu au cours de leur évolution prélever des séquences d'ADN ou d'ARN de leur hôte, et les incorporer à leur propre information génétique. Dans le contexte des protéines virales notamment des protéines d'enveloppes exposées en surface, les séquences peptidiques correspondantes peuvent induire une réaction immunitaire auto-immune, et ainsi perturber le fonctionnement normal de l'organisme. Dans le cas du virus VIH-1 on a identifié de telles séquences. Il existe notamment des similitudes multiples entre la molécule gp120/160 et des protéines immuno-régulatrices. Contrairement à l'idée établie ce n'est pas le peptide au sein de la gp120/160 qui directement induit la pathogénèse chez le sujet infecté, mais c'est la réaction auto-immune dirigée contre ce peptide. En effet l'idée fausse d'une action directe du peptide au sein de la gp120/160 est établie à partir d'expériences in vitro où la concentration de gp120/160 utilisée est très supérieure (d'un facteur 1000) à la concentration existant réellement chez le sujet infecté. Le système in vitro montrant un rôle toxique de l'enveloppe gp120/160 n'est donc pas physiologique. On a démontré la participation des sites de similitude à la pathogénèse induite par le VIH-1, mais dans le cadre général d'une réaction auto-immune cellulaire et humorale, conduisant à une dérèglement immunitaire.

Dans la présente demande sont présentés les peptides nouveaux identifiés qui sont partagés par des molécules immuno-régulatrices et le virus VIH-1, jouant un rôle dans la maladie SIDA de par la réaction auto-immune déclenchée contre eux lors de l'infection. On précisera dans ce qui suit l'originalité et la genèse de la découverte des sites peptidiques et leurs propriétés, les stratégies prophylactiques et thérapeutiques à utiliser en conséquence dans le cadre du SIDA et d'autres applications directes, la mise en pratique de leur utilisation.

Lorsqu'on immunise des sujets séronégatifs contre les antigènes du VIH-1, il se produit une réponse immunitaire cellulaire spécifique avec des cellules tueuses spécifiques. Lorsqu'on immunise de la même façon des sujets séropositifs à un stade relativement avancé, quel que soit le protocole d'immunisation, il est difficile, voire impossible de faire apparaître une immunité spécifique. Il y a donc une barrière suppressive qui est installée chez les sujets infectés par le VIH-1. Pour comprendre la cause de cette barrière d'immuno-suppression, on a recherché les sites viraux pouvant interférer avec le bon déroulement de la réaction immunitaire. Leur recherche a pu être réalisée notamment grâce à un logiciel particulier appelé Automat spécialement développé à cet effet (Copyright JF Zagury).

Plusieurs sites partagés par le VIH-1 et des molécules immuno-régulatrices ont été identifiés, qui témoignent de l'existence d'une réaction auto-immune chez les sujets infectés:

- Identité entre la molécule CD4 et la protéine gp120 du virus VIH-1: SLWDQ (résidus 110-114 dans la souche LAI, résidus 60-64 dans le CD4) (IS 1). Ce peptide SLWDQ a de plus les propriétés remarquables suivantes:

- Il est très conservé dans toutes les souches virales connues.
- Il existe une réaction immunitaire très forte contre des peptides contenant ce site, à la fois humorale et cellulaire (proliférative et tueuse) chez les sujets infectés par le VIH-1 (Exemples 4 et 13). Il existe donc une réaction auto-immune dirigée contre ce site chez les sujets infectés par le VIH-1.
- De plus SLWDQ correspond à un site critique de la molécule CD4 pour l'activation des cellules T car des oligopeptides contenant SLWDQ, en concentration suffisante, inhibent (probablement par compétition avec l'interaction CD4-CMH2) l'activation des cellules T induite par l'anti-gène. Ce site peptidique a donc aussi pour application remarquable de pouvoir induire des états d'immuno-suppression ce qui est utile dans certains cas que nous détaillons plus loin.
- Similitudes entre la molécule gp120 de VIH-1 et la protéine Fas inductrice d'apoptose: VEINCTRPNN : IS 22 (résidus 297-306 du VIH-1 souche LAI) et VEINCTRTQN : IS 23 (résidus 99-108 de la protéine Fas), FYCNST : IS 24 (résidus 388-393 du virus VIH-1 souche LAI) et FFCNST : IS 25 (résidus 117-122 de la protéine Fas). Ces peptides ont de plus les propriétés remarquables suivantes:

- Ils sont très conservés parmi les souches connues de virus VIH-1 surtout les souches d'origine caucasiennes. Le seul pentapeptide INCTR : IS 52 est partagé de manière unique entre Fas et VIH-1 et ce dans toute la banque MIPS.
- Il existe une réponse immunitaire cellulaire dirigée contre des peptides (SVEINCTRPNNNTRKSI et GGEFFYCNSTQL) contenant ces sites chez des sujets infectés par les VIH-1 (DF Nixon et al., AIDS, 1991, 5:1049-1059). On a donc identifié d'autres éléments d'une réaction auto-immune chez les sujets infectés par le VIH-1.
- Ces sites peptidiques correspondent aux 2 seuls sites glycosylés de la molécule Fas et sont aussi potentiellement glycosylés dans le VIH-1. Cela explique que la réponse humorale contre des peptides contenant ces séquences soit relativement faible chez les sujets séropositifs (contrairement à la réponse existant contre les peptides contenant SLWDQ) car dans la molécule native in vivo ces peptides sont associés à des sucres.
- Le virus VIH-2 possède de manière très conservée le site CNST et il partage avec la protéine Fas, et ce de manière unique dans la banque MIPS, l'identité CNSTV : IS 26 (résidus 445-449 dans gp110 de VIH-2, souche ROD).
- Similitude entre le récepteur (chaîne delta) au butyrate et la protéine pol du virus VIH-1: YVGSDLEI (résidus

**EP 0 656 010 B1**

355-362 de VIH-1 souche LAI) et YVGSNLEI (résidus 27-34) du récepteur Fc des IgE. Ce peptide a de plus les propriétés remarquables suivantes:

- Ce site peptidique est bien conservé parmi les différents isolats de VIH-1 connus.
- Il existe une réponse immunitaire cellulaire dirigée contre un peptide (NPDIVIYQYMDDLYVGSDLEIGQHR) contenant ce site chez des sujets infectés par les VIH-1 (DF Nixon et al., AIDS, 1991, 5:1049-1059). On a donc identifié un autre élément d'une réaction auto-immune chez les sujets infectés par le VIH-1.

- Similitudes entre la molécule d'adhésion des leucocytes ELAM-1 et la proteine pol du VIH-1: PLTEEA (résidus 461-466 du VIH-1 souche LAI, et 89-94 de ELAM-1), et QGQWTYQ (résidus 501-507 du VIH-1 souche LAI) avec QGQWTQQ (résidus 350-356 de ELAM-1). Ces peptides ont de plus les propriétés remarquables suivantes:

- Ils sont très conservés dans les isolats VIH-1 connus.
- Il existe une réponse immunitaire cellulaire dirigée contre des peptides (PLTEEAELELAENREILKEPVHGVY et EIQKQGQGQWTYQIYQEPFKNLKTG) contenant ces sites chez des sujets infectés par les VIH-1 (DF Nixon et al., AIDS, 1991, 5:1049-1059). Nous avons donc identifié d'autres éléments d'une réaction auto-immune chez les sujets infectés par le VIH-1.

- Similitude entre la protéine GAG (plus précisément le segment p6) du VIH-1 et la chaîne légère lambda des immunoglobulines: RSGVETTTPSQK (résidus 476-487 de GAG souche LAI) et RAGVETTTPSKQ (résidus 179-190 de la chaîne légère d'immunoglobuline). Ce peptide a de plus la propriété remarquable suivante:

- Ce site peptidique est bien conservé parmi les différents isolats de VIH-1 et se trouve dans la partie constante des chaînes légères. La réaction immunitaire face à ce site chez les sujets infectés est en cours d'étude.

- Identité entre le récepteur Fc de forte affinité aux immunoglobulines de type E et la molécule gp160 du VIH-1: EALKYW (résidus 796-801 dans VIH-1 souche LAI, et résidus 150-155 dans le récepteur Fc). Ce peptide a de plus la propriété remarquable suivante:

- Ce site peptidique est bien conservé parmi les différents isolats de VIH-1 connus. La réaction immunitaire face à ce site chez les sujets infectés est en cours d'étude.

Les sites peptidiques du virus VIH-1 précités peuvent engendrer une réaction auto-immune qui contribue aux désordres immunitaires observés dans le SIDA. Les 2 molécules CD4 et Fas jouent certainement un rôle critique dans le développement de la maladie. On a en effet purifié par colonne d'affinité, à partir de 2 pools de sérums de malades, des anticorps dirigés contre des peptides contenant SLWDQ qui se montrent très immuno-suppressifs (Exemple 5) et causent l'anergie des cellules CD4. Cette anergie contribue à la dérégulation des cytokines observée lors de l'infection poar le VIH-1 (notamment hyperproduction des interférons alpha et gamma). De plus l'existence de cellules tueuses anti-SLWDQ peut contribuer à la délétion des cellules CD4. On a aussi observé l'existence du phénomène de mort par apoptose dans les PBLs de sujets malades, soumis à une activation antigénique in vitro. L'identification des similitudes surprenantes entre Fas et VIH-1 jette une lumière toute nouvelle sur ce phénomène: en effet les PBIs correspondent pour une bonne partie aux cellules mémoires du système immunitaire qui circulent dans l'organisme. Leur mort induite par l'activation par l'antigène montrent qu'elles ont précédemment reçu un signal de mort, et une réponse immunitaire dirigée contre ces segments communs à Fas et à VIH-1 peut expliquer ce phénomène. En particulier les anticorps dirigés contre les sites de glycosylation de Fas sus-cités pourraient agir comme le fait l'anticorps monoclonal APO-1 (Itoh et al., 1991, Cell, 66: 233-245) pour induire la mort programmée des cellules mémoire (dont la prolifération a cessé et qui ne sont donc pas allés au bout de leur différenciation apoptotique induite par lesdits anticorps) lors d'une activation ultérieure. Cliniquement, chez les malades atteints de SIDA il a été observé une relative absence de phénomènes allergiques: une réponse immunitaire dirigée contre le site identité avec le récepteur Fc des IgE peut expliquer cela comme un phénomène de désensibilisation progressive. La réponse immunitaire dirigée contre l'adhésine ELAM-1 peut expliquer des désordres pour le déroulement de la réponse face à des agents pathogènes. Le butyrate est un régulateur important de l'immunité et une réponse dirigée contre le récepteur au butyrate bloquant ou hyperactivant celui-ci peut aussi contribuer aux dérèglements du système immunitaire. Enfin la présence de la similitude importante entre la chaîne légère lambda et le VIH-1 peut contribuer par l'existence d'anticorps dirigés contre ce site à la prolifération polyclonale des lymphocytes B observée dans le SIDA.

Zaïtseva et al. dans Scandinavian Journal of Immunology, vol. 35, N° 3, Mars 1992, pages 267-273, décrit un peptide correspondant aux acides aminés 284-294 de la gp120 et met en relation ce peptide avec la vaccination dans la thérapie du SIDA.

Berzofsky et al. dans The Journal of Clinical Investigation, Vol. 88, N° 3, Septembre 1991, pages 876-884, décrit le peptide HEDIISLWDQSLR en précisant qu'il peut être utilisé pour immuniser des souris en vue de la production de cellules T capables de réagir avec l'enveloppe protéique intacte d'HIV. Cette référence précise encore que ces peptides peuvent de ce fait être utiles à la fois pour développement de vaccins pour la population humaine en général, ainsi que dans le diagnostic ou le pronostic.

Il serait souhaitable de disposer d'armes efficaces pour lutter contre le développement du SIDA. C'est pourquoi la présente invention a pour objet l'utilisation d'un peptide monomère reconnu par une réponse immunitaire humorale ou cellulaire et produisant une réaction auto-immune chez les sujets infectés par VIH-1 ou 2, caractérisé en ce qu'il est choisi dans le groupe constitué par

SLWDQ        (ISN1)
VEINCTRR$^1$R$^2$N (R$^1$=T ou P, R$^2$=N ou Q) (IS N 22 et 23)
FR$^2$CNST (R$^2$=Y ou F) (IS N 24 et 25)
YVGSR$^3$LEI (R$^3$= D OU N) (IS N 27 et 28)
PLTEEA        (IS N 29)
QGQWTR$^4$Q (R$^4$=Y ou Q) (IS N 30 et 31)
RR$^5$GVETTTPS (R$^5$=S ou A) (IS N 48 et 49)
EALKYW (IS N 34)
CNSTV (IS N 26)
VEINCTR (IS N 37)
INCTR (IS N 52)

et en ce qu'il comporte si désiré du côté N terminal et C terminal de 1 à 3 autres acides aminés en enchaînement peptidique, ainsi que ses multimères en séquence linéaire comportant de 2 à 6 unités monomères pour l'obtention d'un médicament destiné à limiter chez l'homme les réactions auto-immunes induites par l'un des virus VIH 1 et 2 ou destiné à induire chez l'homme un état d'immuno-suppression, par exemple dans le cas de transplantations, notamment d'organes. Le nombre total d'acides aminés de la séquence est de préférence supérieur ou égal à 10.

Le monomère d'origine provient de préférence du virus ou de la molécule immuno modulatrice.

Une fois le concept d'auto-immunité face à des sites précisément définis du virus précisé, il est possible de lutter contre cet aspect de la maladie en rendant l'organisme tolérant contre les sites en question. Cela peut se faire classiquement en injectant au sujet les peptides ci-dessus multimérisés par liaison par exemple au mPEG, ou par exemple par traitement au glutaraldehyde, ou encore par simple multimérisation de séquence. Le produit peut être injecté dans un solvant adéquat ou même comme cela a été décrit par exemple dans de l'eau distillée en injection intra-musculaire ou par voie intra-nasale par petites instillations répétées. Ainsi des souris ont été rendues tolérantes contre le peptide DIISLWDQSLKPCVK en leur instillant pendant 5 jours consécutifs une dose de 1 microgramme de peptide dans 20 microlitres d'eau distillée. De manière générale les doses à utiliser sont à adapter au conditions de l'injection (sujet, mode, peptide), mais il est à remarquer que les doses utilisées sont en général inférieures aux doses nécessaires aux immunisations conventionnelles. La tolérance ne dure pas de manière indéfinie et il est préférable de tester l'état immunitaire du sujet traité face à l'épitope considéré: en général il semble convenable de réitérer l'induction de la tolérance toutes les 6-8 semaines au moins dans les premiers 8 mois du traitement. Le produit injecté peut-être un peptide (viral ou de la protéine d'origine notamment), un fragment de protéine (par exemple un fragment de la protéine virale contenant le site et préparée par génie génétique) ou une protéine entière (par exemple la protéine gag produite dans des bactéries recombinantes) contenant les sites de similitude pré-cités, caractérisés en ce qu'ils comportent de 4 à 1000, de préférence de 4 à 200 acides aminés, plus préférentiellement de 4 à 20 acides aminés, notamment 5 à 12 acides aminés ainsi que les dérivés les renfermant. La tolérance peut aussi être induite par injection de liposomes portant des molécules de CMH2 présentant un peptide contenant le site voulu. Pour améliorer l'effet de tolérance les polypeptides utilisés peuvent contenir les séquences voisines dans le virus ou dans la molécule d'origine (CD4, Fas, Récepteur Fc des IgE etc...) du site en question précité. Par exemple les séquences DIISLWDQDIISLWDQ ou DIISLWDQSLKDIISLWDQSLK constituent un dimère de la région virale contenant SLWDQ et peut être utilisée pour provoquer la tolérance ; il en est de même pour la séquence RADSRRSLWDQRADSRRSLWDQ en provenance de la séquence du CD4. De manière générale toute tolérance induite contre les sites précités aide à éviter ou ralentir l'évolution de la maladie SIDA. L'usage de la gp120 toute entière est acceptable, à condition que cela soit sous la forme d'induction d'une tolérance. Cependant il n'est pas recommandé de l'utiliser car parmi les anticorps anti-gp120, dont beaucoup semblent efficaces pour neutraliser les particules virales, tous ne sont pas toxiques sur le plan de l'auto-immunité et il est dommage d'en priver l'organisme. De même l'usage de la molécule CD4 entière pour induire la tolérance est intéressant. Cette molécule CD4 a été préparée sous forme soluble qui permet de l'utiliser facilement.

La tolérance peut également s'obtenir par injection de liposomes portant des molécules CMH de classe II présentant le peptide contre lequel l'organisme doit être rendu tolérant. Pour de meilleurs résultats, il peut être intéressant de multimériser le peptide (par exemple utiliser SLWDQ sous forme de di ou tétramère SLWDQSLWDQ ou SLWDQSLWDQSLWDQSLWDQ. Ce dernier peptide est intéressant car il est suppressif de l'activation des cellules T et il est aussi reconnu par les anticorps des malades infectés par le VIH-1.

La présente demande a de manière générale pour objet tout moyen capable d'empêcher l'obtention d'une réponse immunitaire contre les segments des protéines rétrovirales de VIH-1 et VIH-2, qui présentent une similitude avec des molécules immuno-régulatrices. En particulier les protéines précitées CD4, Fas, récepteur aux IgE, récepteur au butyrate, chaîne légère d'immunoglobuline lambda, ELAM-1.

La tolérance peut également s'obtenir:

- chez le nouveau né en injectant de manière à produire la tolérance de celui-ci à des protéines virales (VIH-1 ou VIH-2) entières ou des fragments de ces protéines, ou plus spécifiquement des peptides contre lesquels on veut induire la tolérance comme ceux précédemment décrits.

On en déduit de ce qui précède qu'il faut éviter la présence des sites précédemment mentionnés dans toute composition vaccinale. Cela peut être fait en utilisant des produits vaccinants (peptides, protéines, ADNs) où les sites en question (en particulier Fas ou CD4) ont été supprimés ou modifiés. Ainsi on a montré que le site SLVDQ n'est pas source d'auto-immunité chez les sujets infectés. Il semble que le tryptophane joue un rôle clé dans ce processus auto-immun (comme il joue un rôle déterminant dans l'activation des cellules T), et donc toute modification du tryptophane (W) en particulier doit être intéressante. Aussi une réponse dirigée contre les zones voisines de ces sites devrait permettre de les neutraliser: par exemple immuniser contre le peptide HEDII (gp120 105-109) ou SLKPCVK (gp120 115-121) ou HEDIISL (gp120 105-11) qui chevauche partiellement SLWDQ.

Beaucoup d'essais de vaccination ont actuellement lieu avec l'enveloppe gp160 entière du virus. Il semble y avoir un réel danger d'auto-immunité induit par de telles immunisations. La présente demande a donc aussi pour objet des molécules d'enveloppe gp160 mutées, additionnées ou délétées d'un ou plusieurs (par exemple 1, 2 ou 3) acides aminés au niveau des sites CD4 et Fas:

- par exemple SLWDQ peut être transformé en SLVDQ, VEINCTR peut être transformé en VEIDCSR, et FYCNST en FYCDSS. Toute modification d'acide(s) aminé(s) évitant une réponse auto-immune est suffisante. Dans le cas de Fas, il est possible que les sites NXT soient une clé majeure dans le phénomène auto-immun car la glycosylation est connue pour son rôle en immunologie et il peut donc être intéressant de les modifier tout particulièrement.

Les peptides et protéines natifs ou modifiés définis ci-dessus, ainsi que les compositions pharmaceutiques les renfermant peuvent donc être utilisés à titre de traitement pour créer une immunisation pour lutter contre l'infection par le VIH-1 ou la prévenir. Il peuvent être administrés à titre curatif ou préventif.

Les peptides immunogènes ont une très faible toxicité ; administrés à la dose de 50 μg à la souris, aucune toxicité n'a été observée.

Une composition ci-dessus peut être administrée par n'importe quelle voie conventionnelle en usage dans le domaine des vaccins, en particulier par voie sous-cutanée, par voie intramusculaire, par voie intraveineuse ou par voie intradermique, par exemple sous forme de suspension injectable, ou encore par voie orale selon l'application que l'on veut mettre en oeuvre. L'administration peut avoir lieu en dose unique ou répétée une ou plusieurs fois après une certain intervalle. Le dosage varie en fonction de divers paramètres, par exemple, de l'individu traité ou du mode d'administration. A titre d'exemple, on indique toutefois que, chez des mammifères tel le rat ou la souris ou le singe, on obtient des résultats satisfaisants avec une dose de chacun des peptides des IS N° 1, 2, 3, 4, 5, d'environ 10 à 100 μg /Kg de poids corporel, de manière avantageuse de 30 à 70 μg /kg de poids corporel, de préférence de 45 à 55 μg /Kg de poids corporel, tant présentés sous forme de protéosomes que couplés à des toxoïdes ou d'autres protéines porteuses (KLH ou BSA...) ou qu'émulsionnés.

Les doses les plus faibles sont avantageusement administrées en présence d'adjuvants et/ou à intervalles répétés tandis que les doses les plus fortes peuvent être administrées une seule fois et/ou en absence d'adjuvant.

Ce qui a été dit à propos des sites auto-immuns du VIH-1 s'applique directement au VIH-2. En résumé, pour réaliser une immunisation anti-VIH-2, il est recommandé d'utiliser des protéines de VIH-2 délétées ou modifiées au niveau des sites d'identité avec les protéines CD4 et Fas notamment du site d'identité entre Fas et gp110, CNSTV, précité.

Il semble que les segments glycosylés de la protéine Fas participent comme épitopes cibles à une réaction auto-immune en particulier humorale chez les sujets infectés. En conséquence on peut utiliser certains sucres pour rentrer en compétition avec l'effet de ces anticorps et bloquer le processus auto-immun anti-Fas. Ces sucres peuvent être par exemple des molécules de glucosamine açétylées (GlucNac) ou des noyaux galactoses simples ou des galactosides ou d'autres sucres appropriés.

Les expériences faites sur l'activation des cellules CD4 en présence de peptides contenant SLWDQ montrent le rôle fonctionnel de ce site. Une inhibition dose-dépendante de l'activation des cellules CD4 s'observe à une concentration de l'ordre de 100 microgrammes par millilitre (voir exemple 2). Cette inhibition s'explique par la compétition exercée par le peptide dans l'interaction CD4-CMH2. Dans la structure tri-dimensionnelle connue de la molécule CD4, le site SLWDQ est caché et cela est confirmé par la difficulté à obtenir des anticorps monoclonaux dirigés contre cet épitope, chez la souris. Le fait qu'il y ait compétition avec des peptides contenant la séquence SLWDQ s'explique alors par un changement conformationnel de la molécule CD4 lors de l'activation induite par l'antigène. Ce changement dégagerait la zone SLWDQ, lui permettant par exemple d'interagir avec le CMH2. De la même façon il est logique de penser que les anticorps reconnaissant l'épitope SLWDQ, présents en grand nombre chez les malades pourront bloquer cette interaction comme le fait le peptide: cela contribue à expliquer l'anergie des cellules T observée lors de la maladie SIDA. De la même façon des anticorps dirigés contre le site commun à Fas et gp120 pourront induire une mort programmée des cellules CD4. De ce fait, aussi bien les peptides suppressifs contenant SLWDQ que les anticorps dirigés contre cet épitope ou les épitopes Fas sus-cités peuvent être utilisés pour leurs propriétés immuno-suppressives illustrées ci-après dans la partie expérimentale (dans le cas du CD4) qui justifient leur emploi comme médicaments et leur incorporation dans des compositions pharmaceutiques.

En effet, en ce qui concerne les propriétés immunosuppressives des peptides issus d'une molécule CD4 ci-dessus, ceux-ci, administrés à un mammifère malade entrent en compétition avec les lymphocytes porteurs de CD4, et empêchent la coopération CD4-CMH. En ce qui concerne les anticorps dirigés contre ces peptides, ceux-ci se fixent sur le segment concerné du CD4 et peuvent bloquer aussi son interaction avec le CMH (pouvant conduire à un état d'anergie), ou sinon par action directe sur le CD4 induire des phénomènes de transduction inhibiteurs (anergie) ou activateurs. De même pour les anticorps anti-Fas dirigés contre les épitopes sus-cités qui pourront, utilisés de manière transitoire, bloquer l'activation des cellules T en stimulant leur mort prématurée. Les peptides et anticorps ci-dessus trouvent de ce fait de remarquables applications pour induire des immunosuppressions. Cela s'applique donc notamment aux cas suivants :

- l'immunosuppression dans le traitement des maladies auto-immunes telles que le lupus érythémateux systémique ou la polyarthrite rhumatoïde (pour inhiber l'activation T auto-immune), dans le cas des transplantations, notamment d'organes (on sait d'ailleurs que l'anticorps OKT4A est utilisé avec un certain succès dans ce but), dans le cas des leucémies et particulièrement des leucémies T pour bloquer la prolifération cellulaire.

Il est à souligner que le peptide de la gp120 HEDIISLWDQSLKPCFK (IS 46) qui contient une proline conservée au même niveau dans la molécule CD4 (SLWDQGNF**P**) est l'un des meilleurs inhibiteurs de l'activation des cellules T. Il est donc important de mentionner des peptides contenant cette proline comme le peptide IS 46. On peut associer dans une même molécule des peptides ci-dessus avec d'autres peptides qui renforcent leur action : par exemple, le site CD4-binding de liaison du VIH-1 au récepteur CD4 permettra un meilleur ancrage à la cellule T et ainsi l'action des peptides immunosuppressifs tel HEDIISLWDQSLKPCVK sera renforcée (IS 47). Cette association dans une même molécule peut se faire de multiples façons, par exemple en associant les deux séquences par une autre séquence telle que (G)n, ou en réalisant des structures peptidiques "en candélabres" notamment par des résidus lysine (K)

Les peptides ci-dessus peuvent être utilisés tels quels ou sous forme de dérivés, associés par exemple à des polysaccharides, des peptides, des noyaux. De tels dérivés entrent dans le cadre de la définition des peptides selon l'invention. C'est pourquoi parmi les peptides ci-dessus, on retient notamment ceux comprenant une séquence issue du site de liaison de gp120 avec la molécule CD4, provenant de VIH-1 ou de VIH-2 (résidus 423-450 dans Env VIH-1 LAI) (cf IS8).

Toutes les indications données ci-dessus par référence aux molécules immorégulatrices, notamment le CD4, peptides, anticorps s'appliquent également notamment aux peptides du CMH2 situés dans une zone essentielle pour l'activation des lymphocytes T dans la réponse immunitaire. En effet, en se basant notamment sur les données cristallographiques obtenues pour le CMH1, on a identifié une région du CMH2, chaîne β, qui est exposée spatialement et qui est susceptible d'intervenir dans la liaison avec le CD4. La séquence de cette région est : FLNGQEETAGWSTN (IS 16).

On a montré expérimentalement que les peptides issus de ce site sont immunosuppressifs (voir exemple 7) comme le sont les peptides CD4 déjà décrits.

Comme pour le CD4 on pourra aussi obtenir les éléments suivants :

- peptides utilisés tels que ou couplés comme agents immunosuppresseurs ;
- anticorps reconnaissants l'épitope CMH2 ci-dessus et induisant ainsi un effet d'immunosuppression.

Les antigènes polypeptidiques ci-dessus décrits (peptides, fragments de protéines, protéines) et leurs dérivés, peuvent être préparés selon des méthodes connues en elles-mêmes, par exemple par synthèse chimique classique pour les peptides (on peut pour cela par exemple opérer une synthèse en phase solide àpartir d'un support solide aminé et des acides aminés désirés), par purification biochimique appropriée ou génie génétique (systèmes eucaryotes ou procaryotes au choix selon les contraintes biologiques requises telle la glycosylation) pour les fragments de protéines du VIH-1 ou des molécules immuno-régulatrices, ou pour ces protéines elles-mêmes. Par "dérivés renfermant lesdits peptides" l'on entend par exemple les structures protéiques, glycoprotéiques ou peptidiques renfermant ledit peptide, et destinées à optimiser l'utilisation désirée du peptide: par exemple il semble que le peptide multimère SLWDQSLWDQSLWDQSLWDQ donne de meilleures suppressions que le peptide SLWDQ seul.

Les immunisations se font classiquement avec des quantités d'antigènes (que ce soient des peptides, polypeptides, fragments de protéines) allant de 10 μg à 20 mg mais il est possible de donner de plus fortes ou de plus faibles doses.

On sait qu'en thérapie humaine, les antigènes peuvent être utilisés directement comme immunogène dans des émulsions eau dans huile ou enchâssés au sein d'adjuvants aqueux de phosphate de calcium ou encore adsorbés sur un gel de phosphate d'alumine. Les antigènes peuvent aussi être présentés conjugués à des protéines porteuses, comme le toxoïde tétanique ou encore le KLH. Enfin, les antigènes peuvent également être présentés au sein de bactéries dans des constructions de recombinaison génétique, après l'insertion de séquences nucléotidiques appropriées dans des plasmides.

Une composition pharmaceutique renfermant de tels antigènes est avantageusement destinée à la prévention ou au traitement d'une infection à VIH-1.

Une composition selon l'invention peut être administrée par n'importe quelle voie conventionnelle en usage dans le domaine des vaccins, en particulier par voie sous-cutanée, par voie intramusculaire, par voie intraveineuse ou par voie intradermique, par exemple sous forme de suspension injectable, ou encore par voie orale selon l'application que l'on veut mettre en oeuvre. L'administration peut avoir lieu en dose unique ou répétée une ou plusieurs fois après une certain intervalle.

Le dosage varie en fonction de divers paramètres, par exemple, de l'individu traité ou du mode d'administration. A titre d'exemple, on indique toutefois que, chez des mammifères immunisés comme des souris ou des singes, on obtient des résultats satisfaisants avec une dose de chacun des peptides des IS N° 1, 2, 3, 4, 5, d'environ 10 à 100 μg /Kg de poids corporel, de manière avantageuse de 30 à 70 μg /kg de poids corporel, de préférence de 45 à 55 μg /Kg de poids corporel, tant présentés sous forme de protéosomes que couplés à des toxoïdes ou d'autres protéines porteuses (BSA, KLH...) ou qu'émulsionnés.

Les doses les plus faibles sont avantageusement administrées en présence d'adjuvants et/ou à intervalles répétés tandis que les doses les plus fortes peuvent être administrées une seule fois et/ou en absence d'adjuvant.

Des immunisations peuvent aussi être réalisées directement par injection d'ADN correspondant aux protéines ou fragments de protéines qui nous intéressent (cf paragraphes précédents) et qui est capté par les cellules et produit localement.

De manière générale toute méthodologie aboutissant à la création d'une réponse immunitaire humorale (anticorps) ou cellulaire (cellules effectrices) sera valable.

Les produits ci-dessus sont dotés de remarquables propriétés qui justifient leur utilisation comme médicament.

L'usage de ces peptides ou dérivés dans les tests, in vitro, notamment de type ELISA pour évaluer l'évolution de la maladie, sachant que de fortes réponses en ELISA face à ces peptides peuvent être corrélées à une forte défaillance de l'immunité. De tels peptides sont notamment DIISLWDQSLK (IS3) ou TASQK (IS12) qui présentent des réponses extrêmement élevées chez certains malades (voir Exemple 4). Des tests de diagnostic en découlent.

La présente demande a en résumé comme objets:

- l'utilisation d'un peptide monomère reconnu par une réponse immunitaire humorale ou cellulaire et produisant une réaction auto-immune chez les sujets infectés par VIH-1 ou 2, caractérisé en ce qu'il est choisi dans le groupe constitué par

SLWDQ        (IS N° 1)
VEINCTRR$^1$R$^2$N (R$^1$=T ou P, R$^2$=N ou Q) (IS N°22 et 23)
FR$^2$CNST (R$^2$=Y ou F) (IS N° 24 et 25)
YVGSR$^3$LEI (R$^3$= D OU N) (IS N° 27 et 28)
PLTEEA        (IS N° 29)
QGQWTR$^4$Q (R$^4$=Y ou Q) (IS N° 30 et 31)
RR$^5$GVETTTPS (R$^5$=S ou A) (IS N° 48 et 49)
EALKYW (IS N° 34)
CNSTV (IS N° 26)
VEINCTR (IS N° 37)
INCTR (IS N° 52)

et en ce qu'il comporte si désiré du côté N terminal et C terminal de 1 à 3 autres acides aminés en enchaînement peptidique, ainsi que ses multimères en séquence linéaire comportant de 2 à 6 unités monomères, pour l'obtention d'un médicament destiné à limiter chez l'homme les réactions auto-immunes induites par l'un des virus VIH 1 et 2 ou destiné à induire chez l'homme un état d'immuno-suppression, par exemple dans le cas de transplantations, notamment d'organes,

- d'un peptide tel que défini ci-dessus, caractérisé en ce que le monomère d'origine est viral ou provient d'une molécule immunorégulatrice.
- d'un peptide, fragment de protéine ou protéine renfermant un peptide tel que défini ci-dessus caractérisé en ce que ledit peptide est modifié par délétion, addition ou modification d'un ou plusieurs acides aminés pour perdre son caractère d'épitope chez les sujets infectés par VIH-1 ou VIH-2.
- d'un peptide comportant de 5 à 20 acides aminés, caractérisé en ce qu'il est d'origine virale, en ce qu'il est adjacent à un des peptides définis ci-dessus dans la séquence virale, et en ce que, du côté C terminal ou N terminal, il chevauche 1 ou 2 acides aminés d'un peptide tel que défini ci-dessus.
- une méthode d'induction d'un état d'immuno-suppression dans lequel on administre par voie parentérale l'un au moins des anticorps définis ci-dessus ;
- une méthode de lutte contre l'infection à VIH-1 ou 2 dans laquelle l'on administre sous forme immunogénique l'un au moins des anticorps définis ci-dessus ;
- et enfin une méthode de lutte contre l'infection à VIH-1 ou 2 dans laquelle l'on administre par voie parentérale l'un au moins des anticorps antiidiotypiques définis ci-dessus.

Les exemples qui suivent illustrent la présente demande

**EXEMPLE 1**

On prépare classiquement du peptide SLWDQ (IS1) et du peptide HEDIISLWDQSLK (IS9) inclus dans des protéosomes. On injecte aux souris par voie intramusculaire 50 µg du produit couplé aux jours J0, J30, J60. On prélève le sérum à J70 pour faire des ELISA. Les résultats obtenus sont les suivants en réponse ELISA par rapport à des sérums témoins de souris non immunisées et à des peptides témoins.

Les souris de la série 1 ont été immunisées par SLWDQ (IS1), les souris de la série 2 ont été immunisées par HEDIISLWDQSLK (IS9), les souris de la série 3 sont des témoins non immunisés.

| Peptide | Souris 1 | Souris 2 | Souris 3 |
|---------|----------|----------|----------|
| IS1 | +++ | +++ | - |
| IS10 | - | - | - |
| IS11 | - | - | - |
| IS9 | +++ | +++ | - |

Les résultats montrent que les peptides IS1 et IS9 sont immunogènes et on a pu obtenir des anticorps contre ces peptides par immunisation chez la souris. L'exemple 4 ci-après montre d'ailleurs l'existence d'anticorps dirigés contre ces peptides chez les malades infectés.

**EXEMPLE 2** : Inhibition par des peptides suppresseurs de l'activation des cellules T.

- Des cellules mononuclées du sang frais de sujets non infectés (PBMC) ont été isolées par Ficoll-Hypaque. Les monocytes macrophages ont été ensuite purifiés par adhérence sur plastique. Les cellules adhérentes ont été incubées 6 heures à 37° avec 1µg/ml de SEB (Entérotoxine B de staphylocoque ou de la tuberculine (PPD) Mérieux à 0,02%. Après lavage, ces cellules ont été utilisée comme cellules présentatrices. Des cellules T répondeuses ont été obtenues par sélection immunomagnétique négative : les cellules PBMC ont été incubées avec l'anticorps monoclonal anti-HLA-DR et lavées puis mélangées avec des billes recouvertes par des anticorps anti-(IgG de souris).
  Les cellules HLA DR (+) (cellules déjà activées) ont été enlevées avec un aimant pendant 5 mn. Les cellules HLA DR (-) (cellules au repos) ont ainsi pu être récupérées.
- Des cellules autologues présentatrices sont cultivées en mélange avec des cellules répondeuses HLA DR (-) avec différents peptides pendant 5 jours à 37°. Les réponses en prolifération sont mesurées par l'incorporation de thimidine tritiée. Les résultats consignés dans le tableau suivant montrent l'effet inhibiteur des peptides contenant la séquence SLWDQ (IS1). Ces expériences montrent aussi le rôle inhibiteur des peptides issus du site de liaison de la gp120 avec le CD4. Le site de liaison du CD4 avec la gp120 (résidus 40-59 du CD4) pourra lui aussi être inhibiteur.

| PEPTIDES (souche LAI) | | Prolifération des lymphocytes T | |
|---|---|---|---|
| | | PPD | SEB |
| 1. HEDIISLWDQSLK | env 105-117 | 4320 ± 1500 | 14670 ± 4470 |
| 2. SLWDQ (IS1) | env 110-114 | 5500 ± 1780 | 12330 ± 3280 |
| 3. SLWDQSLKPCVKLTPL | env 110-125 | 6070 ± 1580 | 16760 ± 2530 |
| | | | |
| 4. IRIQRGPGRAF | env 312-320 | 12200 ± 2370 | 27180 ± 5360 |
| 5. KQIINMWQEVGKAMYA | env 429-440 | 6650 ± 1880 | 15130 ± 3430 |
| 6. CRIKQINMWQEVKAMY APPISGQIR | env 423-447 | 5780 ± 2110 | 14210 ± 2770 |
| 7. SSGGDPEIVTHSFNC | env 369-383 | 14180 ± 2300 | 33360 ± 3460 |
| 8. gp120 | | 3400 ± 1220 | 10230 ± 2710 |
| No peptide | 1 | 5400 ± 1900 | 31990 ± 780 |

## EXEMPLE 3

On immunise des souris, comme dans l'exemple 1, par le peptide HEDII (IS5) qui est adjacent à SLWDQ (IS1) dans le VIH-1 (séquence 105-109).

On utilise les anticorps de souris dans des expériences d'inhibition décrites à l'exemple 2 : on arrive à diminuer l'effet inhibiteur de la gp120 grâce à ces anticorps.

En effet, au lieu d'une inhibition de 80% en présence de 300 µg de gp120, on voit une inhibition de seulement 50% en présence d'anticorps : ces anticorps diminuent l'effet suppresseur de la gp120.

| | Prolifération des lymphocytes T | |
|---|---|---|
| | PPD | SEB |
| gp120 | 3400 | 10230 |
| gp120 + anticorps | 8220 | 17450 |
| témoin | 15400 | 31900 |

## EXEMPLE 4

Des tests ELISA ont été faits à partir des sera de 14 sujets séronégatifs (contrôle), 14 sujets infectés par le VIH-1 asymptomatiques, 14 sujets atteints de SIDA.

Les Densités optiques (D.O.) obtenues par ELISA ont été rapportées au sérum non dilué. Pour chaque groupe, la moyenne des réponses obtenues pour chaque peptide a été calculée. Le tableau suivant présente les rapports des moyennes obtenues pour les groupes des sujets infectés asymptomatiques ou SIDA sur les moyennes obtenues pour les séronégatifs.

On note les fortes réponses anticorps contre DIISLWDQSLK (IS3) et contre TASQK (IS12) par rapport aux autres peptides.

| PEPTIDES | ASYMPTOMATIQUES | SIDA |
|---|---|---|
| SSGGDPEIVTHSFNC (Env 365-383 VIH-1 LAI) | 2.5 | 2 |

(suite)

| PEPTIDES | ASYMPTOMATIQUES | SIDA |
|---|---|---|
| KAKRRVVQREKRAVG (Env 505-519 VIH-1 LAI) | 2.4 | 2.1 |
| SLWDQ (IS1) (Env 101-117 VIH-1 LAI) | 3.5 | 3 |
| DIISLWDQSLK (IS3) (Env 107-117 VIH-1 LAI) | 20 | 11 |
| NMWQEVGKAMYA (Env 430-441 VIH-1 LAI) | 1.9 | 1.5 |
| TASQK (IS12) (CD4 17-21) | 5 | 4 |

**EXEMPLE 5** : Purification d'anticorps à spécificité anti-SLWDQ (IS1) et leur effet immunosuppresseur.

Les sérums des souris immunisées comme décrit à l'exemple 1 par les peptides IS 9 (ou IS10) est incubé avec des billes AFFIGEL 102 (Biorad) à concentration appropriée (par exemple 2 mg de peptide pour 1 mg de gel). On ajoute lors de l'éthanol acétate (agent couplant) et on amène alors le pH à 4,8. On laisse incuber 3 heures à la température ambiante, puis on centrifuge le tout 10 minutes. On rince le gel couplé obtenu en culot avec du PBS + NaN$_3$ à 0,01 %.

Une fois filtré, on peut éluer les anticorps fixés par du tampon acide citrique 0,1 M pH 3. On neutralise la fraction éluée en tampon Tris HCl à pH 9 et on concentre le tout par centrifugation à travers une colonne Centrisart (Seuil = 10 kD).

On vérifie le fort titre en anticorps anti (peptide IS9 ou IS 10) dans la fraction concentrée ainsi recueillie par ELISA.

En réalisant les mêmes expériences d'inhibition que celles décrites dans l'exemple 2 en utilisant ces anticorps purifiés, on obtient une inhibition significative de la prolifération des lymphocytes T activés par les antigènes SEB ou PPD seulement pour les anticorps sélectionnés sur la colonne IS9 (peptide contenant SLWDQ (IS1)) et non pas sur les anticorps sélectionnés sur la colonne couplée avec le peptide IS10.

Les résultats (% d'inhibition) sont décrits dans le tableau suivant (en fonction de la dilution des anticorps):

| DILUTION | 1/25 | 1/50 | 1/100 | 1/150 | 1/200 |
|---|---|---|---|---|---|
| Anticorps anti IS 9 | 100 % | 100 % | 100 % | 80 % | 50 % |
| Anticorps anti IS 10 | 40 % | 0 % | 0 % | 0 % | 0 % |

De la même façon on a purifié des anticorps anti-IS1 chez des sujets séronégatifs et des sujets séropositifs, infectés par le VIH-1.

Typiquement, pour un éluat concentré final de volume 1 ml obtenu par filtration de 5 ml de sérum, les résultats d'inhibition (%) en fonction de la dilution de l'anticorps sont les suivants :

| DILUTION | 1/25 | 1/50 | 1/100 | 1/150 | 1/200 |
|---|---|---|---|---|---|
| Anticorps de séropositif | 100 % | 100 % | 100 % | 90 % | 50 % |
| Anticorps de séronégatif | 90 % | 30 % | 0 % | 0 % | 0 % |

On voit donc qu'il existe typiquement beaucoup plus d'anticorps suppressifs chez le sujet infecté que chez le sujet séronégatif.

**EXEMPLE 6** : Inhibition de l'effet immunosuppressif des anticorps de patient infecté dirigés contre l'épitope SLWDQ (IS1) par un peptide contenant SLWDQ (IS1).

On réalise une expérience de suppression comme celle décrite dans l'exemple 5 avec des anticorps de sujets séropositifs purifiés sur une colonne i couplée avec le peptide IS1 (SLWDQ), ces anticorps étant utilisés dilués au centième. Les résultats d'inhibition obtenus dans une expérience typique, montrent que le peptide (à concentration suffisante) peut neutraliser l'effet de l'anticorps sont donnés dans le tableau ci-après.

| Quantité de peptide présent (en µg/ml) | 10 | 20 | 40 | 60 | 100 |
|---|---|---|---|---|---|
| Inhibition obtenue en présence d'anticorps suppressifs au 1/100e et de peptide | 100 % | 100 % | 100 % | 50 % | 20 % |

**EXEMPLE 7**

Réponse cellulaire tueuse contre certains peptides chez des malades infectés par le VIH-1.

Des cellules mononuclées du sang de malades (PBLs) sont cultivées pendant 1 semaine en présence du peptide stimulant : HEDIISLWDQSLK, à la concentration de 10 µg/ml dans du milieu RPMI sérum de veau foetal 10% supplémenté avec glutamine, antibiotique et IL2 de manière standard, (cellules effectrices : CE)

Après une semaine, on opère le test d'activité tueuse comme suit. On incube les CE avec des cellules autologues présentant le peptide cible à tester. Les cellules autologues sont préparées comme suit : ce sont des cellules B provenant du même malade et transformées par l'EBV. Ces cellules ont été préalablement incubées pendant une heure avec le peptide à tester (concentration 10 µg /ml) pour pouvoir présenter le peptide testé aux cellules effectrices du malade. (ce sont les cellules cibles : CC). Ces cellules sont ensuite incubées avec du $^{51}$Cr pendant une heure, puis lavées.

Pour effectuer le test de lyse 5000 CC sont mélangées pendant quatre heures avec des CE en proportion de 1/30, 1/10, 1/3 et 1/1. La quantité de chrome relarguée dans le surnageant est mesurée dans un compteur gamma et la lyse est calculée comme suit :

$$\frac{\text{Lyse expérimentale (LE) - Lyse spontanée (LS)}}{\text{Lyse totale (LT) - lyse spontanée}}$$

LE = taux de chrome obtenu dans le surnageant expérimental

LS = taux de chrome obtenu avec des CC sans CE

LT = taux de chrome obtenu par lyse totale des CC par l'acide chlorhydrique.

Les résultats obtenus montrent que l'épitope SLWDQ (IS$_1$) est un épitope cible de la réponse immunitaire chez les sujets infectés par le VIH-1. Il en est de même pour d'autres peptides contenant SLWDQ comme IS 46.

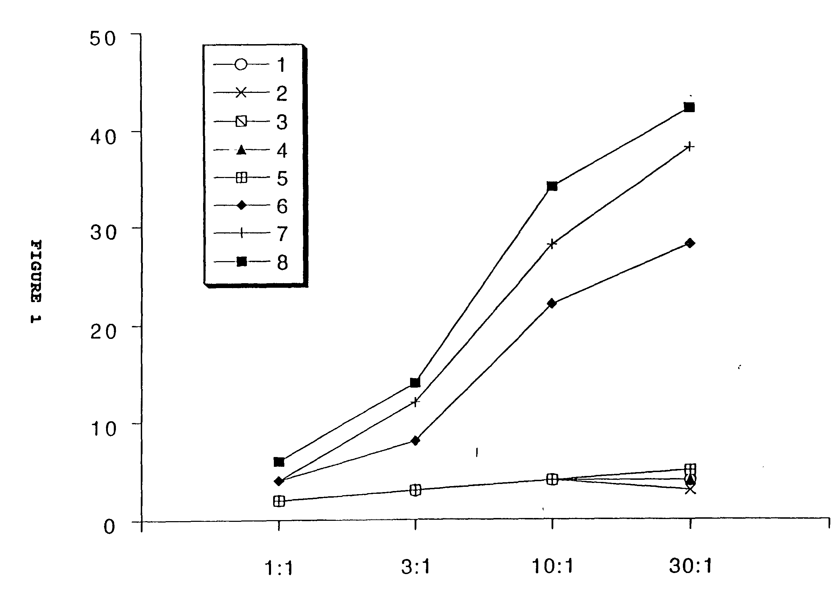

Dans la figure 1 montrant le pourcentage de lyse spécifique en fonction du rapport CE:CC, les peptides testés sont les suivants : P1=IS 6, P2= DWQLS (contrôle négatif), P3=IS 5, P4=IS 35, P5=IS 16, P6=IS 46, P7=IS 1, P8=contrôle positif de vaccine recombinante exprimant l'enveloppe gp160 du virus VIH-1 souche LAI.

Pour les séquences ci-dessous, les numérotations sont faites par rapport à la souche LAI ou bien HXB2 (G. Myers et al 1991).

IS1) Peptide VIH-1 : enveloppe 110-114 (5 acides aminés)

IS2) Peptide CD4 : (CD4 65-69) (5 acides aminés)

IS3) Peptide donnant une réponse ELISA très forte chez les sujets infectés par le VIH-1 : enveloppe 107-117 (11 acides aminés)

IS4) Peptide adjacent à SLWDQ (IS1) : enveloppe 115-125 (11 acides aminés)

IS5) Peptide VIH-1 : enveloppe 105-109, adjacent à SLWDQ (IS1) (5 acides aminés)

IS6) Peptide homologue à SLWDQ (IS1) où le W a été changé en V (5 acides aminés). Ce peptide perd de son activité inhibitrice dans le test de suppression.

IS7) Peptide VIH-1 contenant SLWDQ : enveloppe 105-114 (10 acides aminés).

IS8) Peptide contenant à la fois une partie active de l'enveloppe pour la liaison avec le CD4 (voir exemple 2) et le peptide IS7 suppresseur, séparés par des résidus intermédiaires, par exemple GGG (Enveloppe (426-441) et Enveloppe (105-107) :

      KQIINMWQEVGKAMYAGGGHEDIISLWDQSLK

IS10) Peptide homologue à la région adjacente de SLWDQ dans gp120 (résidus

105-109 : peptide IS5 cité ci-dessus) :

      HEDLQ

IS11) Peptide situé au voisinage du site SLWDQ (IS1) dans la molécule CD4 (résidus 52-60)

      NDRADSRRS

IS12) (CD4 17-21) (5 acides aminés)

IS17) Peptide issu de CD4 contenant le site SLWDQ (IS1) et pour lequel les taux de réponse ELISA sont très élevés chez les sujets infectés par rapport au sujets non infectés :
   ADSRRSLWDQGNFPL (résidus 55 à 69).

IS22 : VEINCTRPNN (résidus 297-306 de gp120, de VIH-1 souche LAI)
IS23 : VEINCTRTQN (résidus 99-108 de la protéine Fas).
IS24 : FYCNST (résidus 388-393 de VIH-1 gp120, souche LAI)
IS25 : FFCNST (résidus 117-122 de la protéine Fas)
IS26 : CNSTV (résidus 119-123 de la protéine Fas)
IS27 : YVGSDLEI (résidus 355-362 de pol de VIH-1, souche LAI)
IS28 : YVGSNLEI (résidus 27-34 du récepteur haute affinité, aux IgE)
IS29 : PLTEEA (résidus 461-466 de pol de VIH-1, souche LAI)
IS30 : QGQWTYQ (résidus 501-507 de pol de VIH-1, souche LAI)
IS31 : QGQWTQQ (résidus 350-356 de ELAM-1)
IS32 : RSGVETTTPSQK (résidus 476-487 de Gag, souche LAI)
IS33 : RAGVETTTPSKQ (résidus 179-190 de la chaîne légère lambda des immunoglobulines)
IS34 : EALKYW (résidus 796-801 de gp120 de VIH-1, souche LAI)
IS35 : SLKPCVK (résidus 115-121 de gp120 de VIH-1, souche LAI)
IS36 : HEDIISL (résidus 105-111 de gp120 de VIH-1, souche LAI)
IS37: VEINCTR (résidus 99-105 de la protéine Fas)
IS38: VEIDCSR (peptide IS37 modifié)
   IS39: FYCDSS (peptide IS24 modifié)
IS40 : HEDIISLVDQSLK (peptide IS9 modifié)
IS41 : DIISLWDQDIISLWDQ (résidus 107-114 de gp120 de VIH-1, souche LAI, dimère)
IS42 : DIISLWDQSLKDIISLWDQSLK (peptide IS3 dimérisé)
IS43 : RADSRRSLWDQRADSRRSLWDQ (peptide 54-64 de CD4, dimère)
IS44 : SLWDQSLWDQ (peptide IS1 dimérisé)
IS45 : SLWDQSLWDQSLWDQSLWDQ (peptide IS1, quadrimérisé)
IS48 : RSGVETTTPS (résidus 476-485 de Gag de VIH-1 souche LAI)
IS49 : RAGVETTTPS (résidus 179-188 de la chaîne légère lambda des immunoglobulines)
IS52 : INCTR (résidus 101-105 de la protéine Fas, communs avec la protéine gp120 de VIH-1)

LISTE DE SEQUENCES

(1) INFORMATION GENERALE:

   (i) DEPOSANT:

      (A) NOM: ZAGURY JEAN-FRANCOIS
      (B) RUE: 117 RUE VIEILLE DU TEMPLE
      (C) VILLE: PARIS
      (E) PAYS: FRANCE
      (F) CODE POSTAL: 75003

   (ii) TITRE DE L' INVENTION: Nouveaux peptides, anticorps diriges contre ces peptides, moyens de blocage de ces anticorps, application a titre de medicaments, compositions pharmaceutiques et methodes d'utilisation.

   (iii) NOMBRE DE SEQUENCES: 52

   (iv) FORME LISIBLE PAR ORDINATEUR:

      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: Patentin Release #1.0, Version #1.25 (OEB)

(2) INFORMATION POUR LA SEQ ID NO: 1:

(i) CARACTERISTIQUES DE LA SEQUENCE:

    (A) LONGUEUR: 5 acides aminés
    (B) TYPE: acide aminé
    (C) NOMBRE DE BRINS: simple
    (D) CONFIGURATION: linéaire

(ii) TYPE DE MOLECULE: peptide

(xi) DESCRIPTION DE LA SEQUENCE: SEQ ID NO: 1:

Ser Leu Trp Asp Gln
1                 5

(2) INFORMATION POUR LA SEQ ID NO: 2:

(i) CARACTERISTIQUES DE LA SEQUENCE:

    (A) LONGUEUR: 5 acides aminés
    (B) TYPE: acide aminé
    (C) NOMBRE DE BRINS: simple
    (D) CONFIGURATION: linéaire

(ii) TYPE DE MOLECULE: peptide

(xi) DESCRIPTION DE LA SEQUENCE: SEQ ID NO: 2:

Gly Asn Phe Pro Leu
1                 5

(2) INFORMATION POUR LA SEQ ID NO: 3:

(i) CARACTERISTIQUES DE LA SEQUENCE:

    (A) LONGUEUR: 11 acides aminés
    (B) TYPE: acide aminé
    (C) NOMBRE DE BRINS: simple
    (D) CONFIGURATION: linéaire

(ii) TYPE DE MOLECULE: peptide

(xi) DESCRIPTION DE LA SEQUENCE: SEQ ID NO: 3:

Asp Ile Ile Ser Leu Trp Asp Gln Ser Leu Lys
1               5               10

(2) INFORMATION POUR LA SEQ ID NO: 4:

    (i) CARACTERISTIQUES DE LA SEQUENCE:

       (A) LONGUEUR: 11 acides aminés
       (B) TYPE: acide aminé
       (C) NOMBRE DE BRINS: simple
       (D) CONFIGURATION: linéaire

    (ii) TYPE DE MOLECULE: peptide

    (xi) DESCRIPTION DE LA SEQUENCE: SEQ ID NO: 4:

Ser Leu Lys Pro Cys Val Lys Leu Thr Pro Leu
1               5               10

(2) INFORMATION POUR LA SEQ ID NO: 5:

    (i) CARACTERISTIQUES DE LA SEQUENCE:

       (A) LONGUEUR: 5 acides aminés
       (B) TYPE: acide aminé
       (C) NOMBRE DE BRINS: simple
       (D) CONFIGURATION: linéaire

    (ii) TYPE DE MOLECULE: peptide

    (xi) DESCRIPTION DE LA SEQUENCE: SEQ ID NO: 5:

His Glu Asp Ile Ile
1               5

(2) INFORMATION POUR LA SEQ ID NO: 6:

    (i) CARACTERISTIQUES DE LA SEQUENCE:

       (A) LONGUEUR: 5 acides aminés

       (B) TYPE: acide aminé

(C) NOMBRE DE BRINS: simple

(D) CONFIGURATION: linéaire

(ii) TYPE DE MOLECULE: peptide
(xi) DESCRIPTION DE LA SEQUENCE: SEQ ID NO: 6:

Ser Leu Val Asp Gln
1               5

(2) INFORMATION POUR LA SEQ ID NO: 7:

(i) CARACTERISTIQUES DE LA SEQUENCE:

(A) LONGUEUR: 10 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 7:

His Glu Asp Ile Ile Ser Leu Trp Asp Gln
1               5                    10

(2) INFORMATION POUR LA SEQ ID NO: 8:

(i) CARACTERISTIQUES DE LA SEQUENCE:

(A) LONGUEUR: 32 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 8:

Lys Gln Ile Ile Asn Met Trp Gln Glu Val Gly Lys Ala Met Tyr Ala
1               5                    10                   15

Gly Gly Gly His Glu Asp Ile Ile Ser Leu Trp Asp Gln Ser Leu Lys
                    20                    25                    30

(2) INFORMATION POUR LA SEQ ID NO: 9:

    (i) CARACTERISTIQUES DE LA SEQUENCE:

        (A) LONGUEUR: 13 acides aminés
        (B) TYPE: acide aminé
        (C) NOMBRE DE BRINS: simple
        (D) CONFIGURATION: linéaire

    (ii) TYPE DE MOLECULE: peptide

(2) INFORMATION POUR LA SEQ ID NO: 10:

    (i) CARACTERISTIQUES DE LA SEQUENCE:

        (A) LONGUEUR: 5 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 10:

His Glu Asp Leu Gln
 1                    5

(2) INFORMATION POUR LA SEQ ID NO: 11:

    (i) CARACTERISTIQUES DE LA SEQUENCE:

        (A) LONGUEUR: 9 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 11:

Asn Asp Arg Ala Asp Ser Arg Arg Ser

1                      5

(2) INFORMATION POUR LA SEQ ID NO: 12:

    (i) CARACTERISTIQUES DE LA SEQUENCE:

        (A) LONGUEUR: 5 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 12:

Thr Ala Ser Gln Lys

1                      5

(2) INFORMATION POUR LA SEQ ID NO: 13:

    (i) CARACTERISTIQUES DE LA SEQUENCE:

        (A) LONGUEUR: 10 acides aminés
        (B) TYPE: acide aminé
        (C) NOMBRE DE BRINS: simple
        (D) CONFIGURATION: linéaire

    (ii) TYPE DE MOLECULE: peptide

(2) INFORMATION POUR LA SEQ ID NO: 17:

    (i) CARACTERISTIQUES DE LA SEQUENCE:

        (A) LONGUEUR: 15 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 17:

Ala Asp Ser Arg Arg Ser Leu Trp Asp Gln Gly Asn Phe Pro Leu
1                    5                      10                      15

(2) INFORMATION POUR LA SEQ ID NO: 22:

    (i) CARACTERISTIQUES DE LA SEQUENCE:

        (A) LONGUEUR: 10 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 22:

Val Glu Ile Asn Cys Thr Arg Pro Asn Asn
1                    5                      10

(2) INFORMATION POUR LA SEQ ID NO: 23:

    (i) CARACTERISTIQUES DE LA SEQUENCE:

        (A) LONGUEUR: 10 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 23:

Val Glu Ile Asn Cys Thr Arg Thr Gln Asn
1                    5                      10

(2) INFORMATION POUR LA SEQ ID NO: 24:

    (i) CARACTERISTIQUES DE LA SEQUENCE:

        (A) LONGUEUR: 6 acides aminés
        (B) TYPE: acide aminé
        (C) NOMBRE DE BRINS: simple

(D) CONFIGURATION: linéaire

(ii) TYPE DE MOLECULE: peptide

(xi) DESCRIPTION DE LA SEQUENCE: SEQ ID NO: 24:

Phe Tyr Cys Asn Ser Thr
1                     5

(2) INFORMATION POUR LA SEQ ID NO: 25:

(i) CARACTERISTIQUES DE LA SEQUENCE:

(A) LONGUEUR: 6 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 25:

Phe Phe Cys Asn Ser Thr
1                     5

(2) INFORMATION POUR LA SEQ ID NO: 26:

(i) CARACTERISTIQUES DE LA SEQUENCE:

(A) LONGUEUR: 5 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 26:

Cys Asn Ser Thr Val
1                     5

(2) INFORMATION POUR LA SEQ ID NO: 27:

(i) CARACTERISTIQUES DE LA SEQUENCE:

(A) LONGUEUR: 8 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 27:

Tyr Val Gly Ser Asp Leu Glu Ile
1               5

(2) INFORMATION POUR LA SEQ ID NO: 28:

(i) CARACTERISTIQUES DE LA SEQUENCE:

(A) LONGUEUR: 8 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 28:

Tyr Val Gly Ser Asn Leu Glu Ile
1               5

(2) INFORMATION POUR LA SEQ ID NO: 29:

(i) CARACTERISTIQUES DE LA SEQUENCE:

(A) LONGUEUR: 6 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 29:

Pro Leu Thr Glu Ala Ala

1             5

(2) INFORMATION POUR LA SEQ ID NO: 30:

    (i) CARACTERISTIQUES DE LA SEQUENCE:

        (A) LONGUEUR: 7 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 30:

Gln Gly Gln Trp Thr Tyr Gln

1             5

(2) INFORMATION POUR LA SEQ ID NO: 31:

    (i) CARACTERISTIQUES DE LA SEQUENCE:

        (A) LONGUEUR: 7 acides aminés
        (B) TYPE: acide aminé
        (C) NOMBRE DE BRINS: simple
        (D) CONFIGURATION: linéaire

    (ii) TYPE DE MOLECULE: peptide

    (xi) DESCRIPTION DE LA SEQUENCE: SEQ ID NO: 31:

Gln Gly Gln Trp Thr Gln Gln

1             5

(2) INFORMATION POUR LA SEQ ID NO: 32:

    (i) CARACTERISTIQUES DE LA SEQUENCE:

        (A) LONGUEUR: 12 acides aminés
        (B) TYPE: acide aminé
        (C) NOMBRE DE BRINS: simple

(D) CONFIGURATION: linéaire

(ii) TYPE DE MOLECULE: peptide

(xi) DESCRIPTION DE LA SEQUENCE: SEQ ID NO: 32:

Arg Ser Gly Val Glu Thr Thr Thr Pro Ser Gln Lys
1 5 10

(2) INFORMATION POUR LA SEQ ID NO: 33:

(i) CARACTERISTIQUES DE LA SEQUENCE:

(A) LONGUEUR: 12 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 33:

Arg Ala Gly Val Glu Thr Thr Thr Pro Ser Lys Gln
1 5 10

(2) INFORMATION POUR LA SEQ ID NO: 34:

(i) CARACTERISTIQUES DE LA SEQUENCE:

(A) LONGUEUR: 6 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 34:

Glu Ala Leu Lys Tyr Trp
1 5

(2) INFORMATION POUR LA SEQ ID NO: 35:

(i) CARACTERISTIQUES DE LA SEQUENCE:

(A) LONGUEUR: 7 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 35:

Ser Leu Lys Pro Cys Val Lys
1               5

(2) INFORMATION POUR LA SEQ ID NO: 36:

(i) CARACTERISTIQUES DE LA SEQUENCE:

(A) LONGUEUR: 7 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 36:

His Glu Asp Ile Ile Ser Leu
1               5

(2) INFORMATION POUR LA SEQ ID NO: 37:

(i) CARACTERISTIQUES DE LA SEQUENCE:

(A) LONGUEUR: 7 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 37:

Val Glu Ile Asn Cys Thr Arg
1          5

(2) INFORMATION POUR LA SEQ ID NO: 38:

(i) CARACTERISTIQUES DE LA SEQUENCE:

(A) LONGUEUR: 7 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 38:

Val Glu Ile Asp Cys Ser Arg
1          5

(2) INFORMATION POUR LA SEQ ID NO: 39:

(i) CARACTERISTIQUES DE LA SEQUENCE:

(A) LONGUEUR: 6 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 39:

Phe Tyr Cys Asp Ser Ser
1          5

(2) INFORMATION POUR LA SEQ ID NO: 40:

(i) CARACTERISTIQUES DE LA SEQUENCE:

(A) LONGUEUR: 13 acides aminés
(B) TYPE: acide aminé

(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 40:

His Glu Asp Ile Ile Ser Leu Val Asp Gln Ser Leu Lys
1               5                       10

(2) INFORMATION POUR LA SEQ ID NO: 41:

(i) CARACTERISTIQUES DE LA SEQUENCE:

(A) LONGUEUR: 16 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 41:

Asp Ile Ile Ser Leu Trp Asp Gln Asp Ile Ile Ser Leu Trp Asp Gln
1               5                       10                  15

(2) INFORMATION POUR LA SEQ ID NO: 42:

(i) CARACTERISTIQUES DE LA SEQUENCE:

(A) LONGUEUR: 22 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 42:

Asp Ile Ile Ser Leu Trp Asp Gln Ser Leu Lys Asp Ile Ile Ser Leu

1          5          10          15

Trp Asp Gln Ser Leu Lys

20

(2) INFORMATION POUR LA SEQ ID NO: 43:

   (i) CARACTERISTIQUES DE LA SEQUENCE:

      (A) LONGUEUR: 22 acides aminés
      (B) TYPE: acide aminé
      (C) NOMBRE DE BRINS: simple
      (D) CONFIGURATION: linéaire

   (ii) TYPE DE MOLECULE: peptide

   (xi) DESCRIPTION DE LA SEQUENCE: SEQ ID NO: 43:

Arg Ala Asp Ser Arg Arg Ser Leu Trp Asp Gln Arg Ala Asp Ser Arg

1          5          10          15

Arg Ser Leu Trp Asp Gln

20

(2) INFORMATION POUR LA SEQ ID NO: 44:

   (i) CARACTERISTIQUES DE LA SEQUENCE:

      (A) LONGUEUR: 10 acides aminés
      (B) TYPE: acide aminé
      (C) NOMBRE DE BRINS: simple
      (D) CONFIGURATION: linéaire

   (ii) TYPE DE MOLECULE: peptide

   (xi) DESCRIPTION DE LA SEQUENCE: SEQ ID NO: 44:

Ser Leu Trp Asp Gln Ser Leu Trp Asp Gln

1          5          10

(2) INFORMATION POUR LA SEQ ID NO: 45:

(i) CARACTERISTIQUES DE LA SEQUENCE:

(A) LONGUEUR: 20 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 45:

Ser Leu Trp Asp Gln Ser Leu Trp Asp Gln Ser Leu Trp Asp Gln Ser
1 5 10 15

Leu Trp Asp Gln
20

(2) INFORMATION POUR LA SEQ ID NO: 48:

(i) CARACTERISTIQUES DE LA SEQUENCE:

(A) LONGUEUR: 10 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 48:

Arg Ser Gly Val Glu Thr Thr Thr Pro Ser
1 5 10

(2) INFORMATION POUR LA SEQ ID NO: 49:

(i) CARACTERISTIQUES DE LA SEQUENCE:

(A) LONGUEUR: 10 acides aminés
(B) TYPE: acide aminé
(C) NOMBRE DE BRINS: simple
(D) CONFIGURATION: linéaire

**EP 0 656 010 B1**

(ii) TYPE DE MOLECULE: peptide

(xi) DESCRIPTION DE LA SEQUENCE: SEQ ID NO: 49:

$$\text{Arg Ala Gly Val Glu Thr Thr Thr Pro Ser}$$
$$1 \qquad\qquad 5 \qquad\qquad\qquad 10$$

(2) INFORMATION POUR LA SEQ ID NO: 52:

(i) CARACTERISTIQUES DE LA SEQUENCE:

    (A) LONGUEUR: 5 acides aminés
    (B) TYPE: acide aminé
    (C) NOMBRE DE BRINS: simple
    (D) CONFIGURATION: linéaire

(ii) TYPE DE MOLECULE: peptide

(xi) DESCRIPTION DE LA SEQUENCE: SEQ ID NO: 52:

$$\text{Ile Asn Cys Thr Arg}$$
$$1 \qquad\qquad 5$$

**Revendications**

1. Utilisation d'un peptide monomère reconnu par une réponse immunitaire humorale ou cellulaire et produisant une réaction auto-immune chez les sujets infectés par VIH-1 ou 2, choisi dans le groupe constitué par

    SLWDQ    (IS N°1)
    VEINCTRR$^1$R$^2$N (R$^1$=T ou P, R$^2$=N ou Q)    (IS N°22 et 23)
    FR$^2$CNST (R$^2$=Y ou F)    (IS N°24 et 25)
    YVGSR$^3$LEI (R$^3$= D OU N)    (IS N°27 et 28)
    PLTEEA    (IS N°29)
    QGQWTR$^4$Q (R$^4$=Y ou Q)    (IS N°30 et 31)
    RR$^5$GVETTTPS (R$^5$=S ou A)    (IS N°48 et 49)
    EALKYW    (IS N°34)
    CNSTV    (IS N°26)
    VEINCTR    (IS N°37)
    INCTR    (IS N°52),

ledit peptide comportant si désiré du côté N terminal et C terminal de 1 à 3 autres acides aminés en enchaînement peptidique, ainsi que par ses multimères en séquence linéaire comportant de 2 à 6 unités monomères, pour l'obtention d'un médicament destiné à limiter chez l'homme les réactions auto-immunes induites par l'un des virus VIH-1 et 2.

2. Utilisation d'un peptide monomère reconnu par une réponse immunitaire humorale ou cellulaire et produisant une réaction auto-immune chez les sujets infectés par VIH-1 ou 2, choisi dans le groupe constitué par

**29**

SLWDQ        (IS N°1)
VEINCTRR$^1$R$^2$N (R$^1$=T ou P, R$^2$=N ou Q)        (IS N°22 et 23)
FR$^2$CNST (R$^2$=Y ou F)        (IS N°24 et 25)
YVGSR$^3$LEI (R$^3$= D OU N)        (IS N°27 et 28)
PLTEEA        (IS N°29)
QGQWTR$^4$Q (R$^4$=Y ou Q)        (IS N°30 et 31)
RR$^5$GVETTTPS (R$^5$=S ou A)        (IS N°48 et 49)
EALKYW        (IS N°34)
CNSTV        (IS N°26)
VEINCTR        (IS N°37)
INCTR        (IS N°52)

ledit peptide comportant si désiré du côté N terminal et C terminal de 1 à 3 autres acides aminés en enchaînemet peptidique, ainsi que par ses multimères en séquence linéaire comportant de 2 à 6 unités monomères, pour l'obtention d'un médicament destiné à induire chez l'homme un état d'immunosuppression, par exemple dans le cas des transplantations, notamment d'organes.

**3.** Utilisation selon la revendication 1 ou 2 d'un peptide tel que défini à la revendication 1 ou 2, caractérisée en ce que le peptide monomère est d'origine virale ou provient d'une molécule immunorégulatrice.

**4.** Utilisation selon la revendication 1 ou 2 d'un peptide, fragment de protéine ou protéine renfermant un peptide tel que défini la revendication 1 ou 2, caractérisée en ce que ledit peptide est modifié par délétion, addition ou modification d'un ou plusieurs acides aminés pour perdre son caractère d'épitope chez les sujets infectés par VIH-1 ou VIH-2.

**5.** Utilisation d'un peptide comportant de 5 à 20 acides aminés, d'origine virale, adjacent à un des peptides définis à la revendication 1 dans la séquence virale, et chevauchant du côté C terminal ou N terminal, 1 ou 2 acides aminés d'un peptide tel que défini à la revendication 1 ou 2, pour l'obtention d'un médicament destiné à limiter chez l'homme les réactions auto-immunes induites par l'un des virus VIH-1 et VIH-2 ou d'un médicament destiné à induire chez l'homme un état d'immunosuppression par exemple dans le cas des transplantations, notamment d'organes.

**6.** Utilisation d'un peptide tel que défini à la revendication 1 comprenant la séquence SLWDQ, pour l'obtention d'un médicament destiné à l'induction de la suppression immunitaire chez un sujet.

**Patentansprüche**

**1.** Verwendung eines monomeren Peptids, welches eine humorale oder celluläre Imunantwort bewirkt und eine Autoimmunreaktion bei den mit HIV-1 oder -2 infizierten Subjekten erzeugt, ausgewählt aus der Gruppe, bestehend aus:

SLWDQ        (ID Nr. 1)
VEINCTRR$^1$R$^2$N (R$^1$ = T oder P, R$^2$ = N oder Q)        (ID Nr. 22 und 23)
FR$^2$CNST (R$^2$ = Y oder F)        (ID Nr. 24 und 25)
YVGSR$^3$LEI (R$^3$ = D oder N)        (ID Nr. 27 und 28)
PLTEEA        (ID Nr. 29)
QGQWTR$^4$Q (R$^4$ = Y oder Q)        (ID Nr. 30 und 31)
RR$^5$GVETTTPS (R$^5$ = S oder A)        (ID Nr. 48 und 49)
EALKYW        (ID Nr. 34)
CNSTV        (ID Nr. 26)
VEINCTR        (ID Nr. 37)
INCTR        (ID Nr. 52)

wobei das Peptid, wenn gewünscht, auf der N-terminalen und C-terminalen Seite 1 bis 3 andere Aminosäuren in der Peptidkette aufweist und ebenso seiner Multimeren, die in der linearen Sequenz 2 bis 6 Monomereinheiten aufweisen, um ein Medikament herzustellen, das bestimmt ist, beim Menschen die durch eines der HIV-1- oder -2-Viren induzierten Autoimmunreaktionen zu begrenzen.

2. Verwendung eines monomeren Peptids, welches eine humorale oder celluläre Imunantwort bewirkt und eine Autoimmunreaktion

    bei den mit HIV-1 oder -2 infizierten Subjekten erzeugt, ausgewählt aus der Gruppe bestehend aus:

    SLWDQ        (ID Nr. 1)
    VEINCTRR$^1$R$^2$N (R$^1$ = T oder P, R$^2$ = N oder Q)        (ID Nr. 22 und 23)
    FR$^2$CNST (R$^2$ = Y oder F)       (ID Nr. 24 und 25)
    YVGSR$^3$LEI (R$^3$ = D oder N)       (ID Nr. 27 und 28)
    PLTEEA        (ID Nr. 29)
    QGGWTR$^4$Q (R$^4$ = Y oder Q)        (ID Nr. 30 und 31)
    RR$^5$GVETTTPS (R$^5$ = S oder A)        (ID Nr. 48 und 49)
    EALKYW        (ID Nr. 34)
    CNSTV       (ID Nr. 26)
    VEINCTR        (ID Nr. 37)
    INCTR        (ID Nr. 52)

    wobei das Peptid, wenn gewünscht, auf der N-terminalen und C-terminalen Seite 1 bis 3 andere Aminosäuren in der Peptidkette aufweist und ebenso seiner Multimeren, die in der linearen Sequenz 2 bis 6 Monomereinheiten aufweisen, um ein Medikament herzustellen, das dafür bestimmt ist, beim Menschen einen immunsupprimierten Zustand zu induzieren, beispielsweise im Falle von Transplantationen, insbesondere von Organen.

3. Verwendung eines Peptids, definiert wie in den Ansprüchen 1 oder 2, nach Anspruch 1 oder 2, dadurch gekennzeichnet, daß das monomere Peptid von viralem Ursprung ist oder von einem immunregulatorischen Molekül stammt.

4. Verwendung eines Peptids, Proteinfragments oder Proteins, enthaltend ein Peptid, definiert wie nach Anspruch 1 oder 2, nach Anspruch 1 oder 2, dadurch gekennzeichnet, daß das Peptid durch Deletion, Addition oder Modifikation einer oder mehrerer Aminosäuren modifiziert ist, um seinen Epitop-Charakter bei den durch HIV-1 oder -2 infizierten Subjekten zu verlieren.

5. Verwendung eines Peptids, das 5 bis 20 Aminosäuren aufweist, von viralem Ursprung ist, in der viralen Sequenz benachbart zu einem der nach Anspruch 1 definierten Peptide ist und sich an der C-terminalen oder N-terminalen Seite mit 1 oder 2 Aminosäuren eines Peptids, definiert nach Anspruch 1 oder 2, überschneidet, um ein Medikament herzustellen, das dafür bestimmt ist, beim Menschen die durch eines der HIV-1- und -2-Viren induzierten Autoimmunreaktionen zu begrenzen oder ein Medikament herzustellen, das bestimmt ist, beim Menschen einen immunsupprimierten Zustand zu induzieren, beispielsweise im Falle von Transplantationen, insbesondere von Organen.

6. Verwendung eines Peptids definiert nach Anspruch 1, das die Sequenz SLWDQ aufweist, um ein Medikament herzustellen, das bestimmt ist, die Immunsuppression bei einem Subjekt zu induzieren.

**Claims**

1. Use of a monomer peptide recognised by a humoral or cellular immune response and producing an autoimmune reaction in subjects infected with HIV-1 or 2, selected from the group consisting of:

    SLWDQ        (SI No 1)
    VEINCTRR$^1$R$^2$N (R$^1$=T or P, R$^2$=N or Q)        (SI No 22 and 23)
    FR$^2$CNST (R$^2$=Y or F)       (SI No 24 and 25)
    YVGSR$^3$LEI (R$^3$=D OR N)       (SI No 27 and 28)
    PLTEEA        (SI No 29)
    QGGWTR$^4$Q (R$^4$=Y or Q)        (SI No 30 and 31)
    RR$^5$GVETTTPS (R$^5$=S or A)        (SI No 48 and 49)
    EALKYW        (SI No 34)
    CNSTV       (SI No 26)
    VEINCTR        (SI No 37)
    INCTR        (SI No 52),

the said peptide having, if desired, on the N-terminal and C-terminal side, from 1 to 3 other amino acids in a peptide chain, and also consisting of its multimers in a linear sequence having from 2 to 6 monomer units, in order to obtain a medicament intended to limit in humans the autoimmune reactions induced by one of the viruses HIV-1 and 2.

2. Use of a monomer peptide recognised by a humoral or cellular immune response and producing an autoimmune reaction in subjects infected with HIV-1 or 2, selected from the group consisting of

SLWDQ        (SI No 1)
VEINCTRR$^1$R$^2$N (R$^1$=T or P, R$^2$=N or Q)        (SI No 22 and 23)
FR$^2$CNST (R$^2$=Y or F)        (SI No 24 and 25)
YVGSR$^3$LEI (R$^3$=D OR N)        (SI No 27 and 28)
PLTEEA        (SI No 29)
QGQWTR$^4$Q (R$^4$=Y or Q)        (SI No 30 and 31)
RR$^5$GVETTTPS (R$^5$=S or A)        (SI No 48 and 49)
EALKYW        (SI No 34)
CNSTV        (SI No 26)
VEINCTR        (SI No 37)
INCTR        (SI No 52),

the said peptide having, if desired, on the N-terminal and C-terminal side, from 1 to 3 other amino acids in a peptide chain, and also consisting of its multimers in a linear sequence having from 2 to 6 monomer units, in order to obtain a medicament intended to induce in humans a state of immunosuppression, for example in the case of transplants, especially organ transplants.

3. Use according to Claim 1 or 2 of a peptide as defined in Claim 1 or 2, characterised in that the monomer peptide is of viral origin or is derived from an immunoregulatory molecule.

4. Use according to Claim 1 or 2 of a peptide, a protein fragment or a protein containing a peptide as defined in Claim 1 or 2, characterised in that the said peptide is modified by deletion, addition or modification of one or more amino acids in order to lose its epitopic character in subjects infected with HIV-1 or HIV-2.

5. Use of a peptide having from 5 to 20 amino acids, of viral origin, adjacent to one of the peptides defined in Claim 1 in the viral sequence, and overlapping on the C-terminal or N-terminal side either 1 or 2 amino acids of a peptide as defined in Claim 1 or 2, in order to obtain a medicament intended to limit in humans the autoimmune reactions induced by one of the viruses HIV-1 and HIV-2 or a medicament intended to induce in humans a state of immunosuppression, for example in the case of transplants, especially organ transplants.

6. Use of a peptide as defined in Claim 1 comprising the sequence SLWDQ, in order to obtain a medicament intended to induce suppression of the immune reaction in a subject.

FIGURE 1